# EUROPEAN PATENT SPECIFICATION

(11) **EP 0 764 169 B1**
(45) Date of publication and mention of the grant of the patent: **23.09.1998**
(21) Application number: 95923300.8
(22) Date of filing: 09.06.1995
(51) Int. Cl.: C07H 11/00, C07H 11/04, A61K 31/70, A61K 31/715, A61K 31/66, A61K 47/48

(54) **DRUG SALTS**
SALZE VON MEDIKAMENTEN
SELS MEDICAMENTEUX

(30) Priority: 10.06.1994 DK 66794; 13.03.1995 GB 9505021
(43) Date of publication of application: 26.03.1997
(73) Proprietor: DUMEX-ALPHARMA A/S, 2300 Copenhagen S (DK)
(72) Inventor: DYRSTING, Hjarne, DK-2830 Virum (DK); KOCH, Torben, DK-2400 Copenhagen (DK); PETERSEN, Kim, Voulund, DK-2635 Vallensbaek (DK)
(74) Representative: Cockbain, Julian, Dr.
(86) International application number: EP9502254
(87) International publication number: WO9534571

(56) References cited:
- EP-A- 0 211 268
- WO-A-95/07914
- GB-A- 1 227 830

## Description

This invention relates to salts of biologically active organic molecules (drugs) with sugar acids, in particular salts with mono-, di- and oligosaccharide poly-O-sulphonic and poly-O-phosphonic acids.

It is common practice in the pharmaceutical industry to use salt forms of drugs, e:g. salts with physiologically acceptable organic or inorganic acids and bases, such as hydrogen chloride, sulphuric acid, maleic acid, ethanolamine, meglumine and the like. For drugs with basic groups, e.g. amine groups, it is feasible to use salts with organic or inorganic acids.

The drug salts are frequently used in preference to the drug itself, for example because of their higher solubility or greater biotolerability.

Many alkaline drug compounds cause irritation of tissue or mucosa. In particular, high local concentration can cause severe irritation and ulceration of the oesophagus. These compounds may also have an unpleasant taste and accordingly they can be administered provided with a polymeric film coating to delay drug release. Such coatings however add to the cost and complexity of formulation.

The present invention is based upon the finding that drug salts with sugar acids exhibit surprising beneficial properties, in particular enhanced uptake and controlled release properties. In particular, insoluble or poorly soluble drug:sugar acid salts have been found to have a drug release profile which is not undesirably dependent on the pH of the surrounding body fluid, e.g. gastrointestinal fluid.

In our earlier patent application, PCT/DK94/00341, the aminoglycoside salts of one sugar acid, sucrose-octa-O-sulphonic acid (SOS), were described as having beneficial properties, especially for oral treatment of H. pylori related stomach and duodenal ulcers. We have now found that in general the poorly soluble or insoluble salts of water soluble sugar acids with organic drug compounds (as opposed to soluble drug:sugar acid complexes or complexes of organic drugs with insoluble sugar acid resins) also exhibit beneficial properties.

By the term "sugar acids" is meant herein carbohydrates, e.g. cyclitols (such as inositol) or mono-, di-, oligo- and poly-saccharides (such as xylose, fructose, glucose, sucrose, lactose, maltose, cellobiose, trehalose, sorbitol, mannitol and dextran) which carry sulphur or phosphorus oxyacid groups on the carbohydrate (e.g. saccharide) moieties (e.g. esters of oxyacids, such as phosphorus and sulphur oxyacids, with the sugar hydroxyls), which sugar acids, in the case of the polysaccharides (and preferably also the oligosaccharides, disaccharides, monosaccharides and cyclitols), contain a high ratio of acid groups to monosaccharide units, i.e. at least 2:1, preferably at least 3:1. Such sugar acids have been used in various medicaments but, other than in our copending application PCT/DK94/00341, have not previously been proposed for use in forming salts, rather than soluble complexes, with organic drug compounds.

Thus viewed from one aspect the invention provides a water-insoluble or poorly water-soluble therapeutic compound being a mono- to octa-saccharide acid salt of a biologically active organic compound (other than a sucrose-octa-O-sulphonic acid salt of an aminoglycoside), preferably a salt with a polybasic sugar acid, especially a mono or disaccharide poly-O-sulphonic acid.

The sugar acid, ie. the mono- or octa-saccharide acid, used according to the invention is preferably a sulphate or phosphonate ester, particularly a sulphate, of a mono-, di- or trisaccharide, in particular a polyester or perester, i.e. a compound in which more than one and optionally all of the sugar hydroxyls are esterified, preferably a compound carrying at least 2, and especially at least 3 oxy-acid groups per saccharide unit.

The disaccharide sulphonic acids are especially preferred, in particular the sucrose sulphonic acids such as sucrose-octa-O-sulphonic acid (SOS). Monosaccharide polysulphonic or poly-O-phosphonic acids, such as phytic acid, are also preferred.

The drug may be any organic drug compound capable of forming a sugar acid salt, in particular a compound containing an electron donor base group such as a basic nitrogen atom, e.g. an amine, imine or ring nitrogen. The drug compounds preferably contain one or more exposed protonatable amine functionalities, particularly preferably a plurality of such groups. Drug compounds useful in the production of the water-insoluble or poorly water soluble drug:sugar acid salts of the invention include antibacterial agents (in particular tetracyclins, aminoglycosides, glycopeptides, polypeptides and macrolides), antiviral agents, antimycotics, anti-amoebics, anti-allergics (such as antihistamines), analgesics, anxiolytics, sedatives, hypnotics, anti-emetics, anti-migraine agents, anti-motion sickness agents, antidepressants particularly tricyclic antidepressants (such as imipramine, amitriptyline and doxepin), alkaloids, cardioprotective agents (such as calcium antagonists, e.g. diltiazem, and azepine derivatives), adrenergics, anticholinergics, antispasmodics, antianorexics, and muscle relaxants, particularly the tricyclic muscle relaxants (e.g. cyclobenzaprine and benzoctamine).

The drug sugar acid salt has a low water solubility, for example less than 10g/L, preferably less than 1g/L more preferably less than 10⁻²mM/L, in deionized water at ambient temperature. This poor water solubility both imparts favourable biorelease characteristics and facilitates preparation of the salts by precipitation from aqueous solution.

The drug compound may be, and preferably is, polybasic and the sugar acid, as mentioned above, is preferably a poly or perester which is thus also polybasic. Accordingly the drug salt of the invention may contain further physiologically tolerable counterions. In this regard alkali and alkaline earth metal (e.g. sodium, potassium, magnesium and calcium), aluminium and ammonium and countercations derived from organic bases such as ethanolamine, diethanolamine and meglumine are preferred cations, while bromide, chloride, sulphate, maleate, acetate, fumarate, succinate and other physiologically tolerable anions derived from inorganic or organic acids are preferred anions.

In the solid state, the drug salts of the invention are amorphous or crystalline materials, e.g. presented in sterile, for example sterile crystalline, form.

The drug salts of the invention may be formulated together with conventional pharmaceutical carriers or excipients, optionally together with further bioactive agents, in conventional pharmaceutical administration forms, e.g. powders, capsules, tablets, coated tablets, suspensions, dispersions, drops, aerosols, suppositories, plasters, pastes, creams, emulsions, etc. These may be in sterile form.

Accordingly, viewed from a further aspect the invention provides a pharmaceutical composition comprising a biologically active organic compound together with at least one physiologically acceptable carrier or excipient, characterized in that said compound, which is other than a sucrose-octa-O-sulphonic acid salt of an aminoglycoside, is present in the form of a water-insoluble or poorly water soluble salt with a mono- to octa-saccharide acid.

Viewed from a yet further aspect the invention is also concerned with the use of an effective amount of a biologically active organic compound in the manufacture of a medicament for combatting a condition responsive to said compound, characterized in that said compound is administered as a water-insoluble or poorly water soluble salt with a mono- to octa-saccharide acid, with the proviso that said salt is other than a sucrose-octa-O-sulphonic acid salt of an aminoglycoside.

Viewed from a further aspect, the invention also provides a water-insoluble or poorly water-soluble mono- to octa-saccharide acid salt of a biologically active organic compound, other than an aminoglycoside:SOS salt, for use as a therapeutic agent.

Sugar acids are produced by esterification of a mono-, di- or oligo-saccharide with a polybasic sulphur or phosphorus oxyacid (e.g. a phosphorous or phosphoric acid or a sulphur oxyacid such as sulphurous or more preferably sulphuric acid) or an activated analog thereof (such as sulphur trioxide). One or more, and preferably most or all of the sugar's hydroxyl groups are esterified to yield oxygen-attached acid groups such as O-sulphonic acid groups.

Mono-, di- and trisaccharides are particularly prefered.

The sugar acids are known compounds and are discussed for example by Ochi et al in Chem Pharm Bull 28:638-641(1980) and W089/07932 (Niels Bukh A/S).

Preferred among the sugar acids useful according to the present invention is the sulphate octa-ester of sucrose, β-D-fructofuranosyl-a-D-glucopyranoside octakis (hydrogen sulphate), hereinafter referred to as sucrose-octa-O-sulphonic acid or SOS.

SOS may be prepared by sulphating sucrose with sulphur trioxide in pyridine. SOS forms crystalline, water soluble sodium, potassium, caesium, rubidium and ammonium salts as reported by Ochi (supra).

SOS also forms an aluminium salt, C₁₂ H₅₄ Al₁₆ O₇₅ S₈, which is known as sucralfate. This aluminium salt may be prepared by reaction of SOS with aluminium hydroxide (see US-A-3432489 (Chugai)) and is widely used for the treatment of gastric ulcers, its effectiveness being ascribed to the aluminium hydroxide ions which act as acid neutralizers and absorb pepsin and bile salts (see Nagashima in Clin. Gastroenterol 3(Suppl. 2):117-227(1981)).

The use of soluble complexes of certain sugar acids has been proposed in the literature. Thus Koh et al. in US-A-3506642 proposed the use of organic base complexes with heparinic acid as orally active heparinoid complexes, e.g. for use as anticoagulants, and Yoshikawa et al. in J. Pharm Dyn 5: S-69 (1982) proposed the use of macromolecular complexes of bleomycin with dextran sulphate to promote lymphatic bleomycin uptake. By contrast Mihai et al. in Cellulose Chem. Technol. 27: 393-403 (1993) proposed loading insoluble particulate polysaccharide cation exchangers with propranolol to provide a sustained release administration vehicle for propranolol.

The present invention however is concerned not with soluble drug:sugar acid complexes or with insoluble ionexchange particles loaded with drug molecules, but with the insoluble or poorly soluble salts which organic drug compounds can form with soluble sugar acids.

Although no medical uses for sugar acid salts with bioactive organic counterions have been proposed, there have been several suggestions for the coadminstration of sugar acids or their metal salts together with bioactive agents. Thus for example W089/07932 (Niels Bukh A/S) describes sugar acid metal salts as ingredients for a topical preparation for periodontal treatment, EP-A-403048 (Warner Lambert) discloses admixtures of sucralfate with other anti-ulcer agents, and WO92/18143 (Smith Kline Beecham Plc) discloses admixtures of sucralfate with various antibiotics.

The drug salts of the invention are generally suited for use where controlled release of the active drug is desired, e.g. for topical administration especially to mucous membranes or administration, preferably per oral, into the gastrointestinal tract.

A particularly valuable characteristic of the salts according to the invention is their ability to release the drug when placed in an ion containing aqueous environment such as gastrointestinal fluid, essentially it appears as a result of ion exchange with competing cations such as sodium or magnesium that are present in the body fluid.

One particularly suitable group of drug compounds for use according to the invention are the antibiotics, in particular basic nitrogen atom containing antibiotics, such as aminoglycosides, tetracyclins, polypeptides, macrolides and glycopeptides.

One preferred group of antibiotic drugs with which to form the sugar acid drug salts of the invention is the tetracyclin antibiotics, a preferred example being doxycyclin.

For more than 50 years tetracyclins have been used as antibiotics. By the term "tetracyclin" is meant an antibiotic of the class containing the fused tetracyclic ring system of tetracyclin itself: 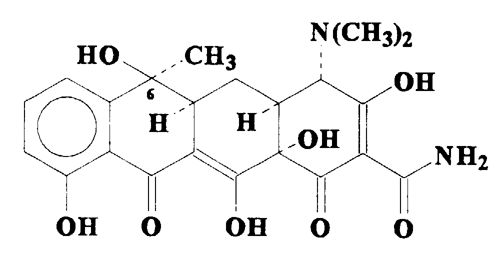 The tetracyclins belong to a group of antibiotics which are manufactured by fermentation of various Streptomyces species. The most widely used are doxycyclin, oxytetracyclin, chlorotetracyclin and tetracyclin. A number of semisynthetic tetracyclins are known, for instance metacyclin and minocyclin. The most widely used of these semisynthetic tetracyclins is α-6-deoxy-5-hydroxy-tetracyclin (doxycyclin). This broad-spectrum antibiotic was first synthesised in 1962 and is marketed by Pfizer under the name Vibramycin®.

Doxycyclin is available in several different forms: doxycyclin monohydrate, doxycyclin hydrochloride (hyclate), doxyclylin carrageenate, doxycyclin calcium and doxycyclin phosphate (fosfatex).

Doxycyclin has a mode of action which is common with other tetracyclins, namely inhibition of bacterial protein synthesis. This arises through inhibition of the binding of aminoacyl-tRNA primarily to 70S ribosomes but also to 30S ribosomes. This results in a bacteriostatic effect. Tetracyclins are active against a broad range of both gram positive and gram negative bacteria, aerobes as well as anaerobes.

Bacterial resistance to tetracyclins is common both in vitro and in vivo, and the resistance is transferred by a plasmid. Due to the bacteriostatic effect of the tetracyclins they usually can not be combined with other cell-cycle specific antimicrobial agents, as the resting cells do not change cell cycle.

Most tetracyclins are incompletely absorbed and their absorption is dependent on the concomitant food intake. Absorption of doxycyclin however is almost complete (73-95%) and independent of food intake (see Saivin et al. in Clin. Pharmacokinetics 15:355-366 (1988)).

The pharmacokinetic parameters of the different salts (hyclate, monohydrate, carrageenate etc.) of doxycyclin do not significantly differ under standard conditions (see Saivin et al. (supra) and Grahnén "Effect of increasing gastric pH on the relative bioavailability of doxycyclin carageenate tablets 100mg (Kabi Pharmacia) in comparison", Internal Study Report, PCB, Sweden, (1991)).

Two factors have been reported which influence the pharmacokinetics of doxycyclin. The pH in the stomach (see Grahnén (supra)) and the concomitant administration of oral antacids (see Nguyen in Antimicrob. Agents Chemother. 33:434-436 (1989)).

An increased pH of the stomach (see Bogardus et al. in J. Pharm Sci 68:1183-1184(1979)) decreases the bioavailability of doxycyclin monohydrate whereas dissociation and absorption of doxycyclin hyclate and doxycyclin carregeenate are independent of pH.

The increased pH in the stomach after omeprazole administration is thus expected to slow down the dissolution of doxycyclin monohydrate and thereby decrease its absorption.

Doxycyclin was first introduced into clinical practice in 1968 as the hydrochloride salt, doxycyclin hyclate. This salt was formulated in tablets or capsules. However, it was soon shown that these formulations had serious side effects. In a study of adverse drug reactions from antibiotics, 35/113 (31%) of patients treated with doxycyclin hyclate after questioning reported nausea and vomiting while 24/373 (6.4%) spontaneously reported nausea and vomiting. These frequencies where 3-fold higher than those reported with other antibiotics.

Another side effect of doxycyclin hyclate is oesophageal ulceration, which can occur if the capsules for some reason do not reach the stomach but remain in the oesophagus.

A solution to these problems has been attempted by the introduction of doxycyclin hydrate (base). This new formulation eliminated the above mentioned side effects, but it soon became apparent that the bioavailability in a number of patients, which were also in treatment with antacids and the like, was significantly reduced. This can be explained by the lack of acid production in the stomach being the cause of reduced dissolution of doxycylin hydrate.

Considering that a great deal of the population has elevated gastric pH caused by either achlorhydria or due to the intake of antacids, H2-blockers, omeprazole or the like, antibiotic treatment with doxycyclin hydrate gives an unacceptably low bioavailability.

One solution to this problem has recently been suggested by the introduction of doxycylin carrageenate, which has a satisfactory bioavailability in subjects with elevated gastric pH. In subjects with normal pH conditions in the stomach, the use of doxycyclin carrageenate has no advantage due to the spontaneous cleavage of doxycyclin carrageenate into doxycyclin H⁺ and carrageenate ion.

By using different pharmaceutical preparations of doxycyclin, attempts have been made to achieve a controlled release effect.

One solution has been the use of film coated tablets. A coated doxycyclin hyclate formulation was developed which showed less tendency to disintegrate in the oesophagus (see Delphre et al. in Digestive Disease and Sciences 34:197-800(1989)). An enteric coated pellet formulation of doxycyclin (sold under the names Doryx® and Doxylets®) has been developed to prevent the total dose of doxycyclin hyclate dissolving in a small area of the stomach. Such formulations have been shown to have a reduced (approximately 50% reduction) rate of nausea and vomiting and an unchanged bioavailability. A pellet formulation however does not automatically have an unchanged bioavailability. In a doxycyclin pellet formulation developed at the university of Nanking, China, it was found that 200mg of the pellet formulation were bioequivalent to 100mg of the standard doxycyclin hyclate formulation (see Qiu et al. in Acta Pharm. Sinica 21:370-376(1986)). Therefore a need still exists for tetracyclin and particularly doxycyclin formulations with controlled release properties.

In EP-A-91409 (Kabi Vitrum) complexes between carrageenan and three drug compounds are described. Carrageenan is a sulphated polysaccharide with a molecular weight from 100kD to 1000kD which carries a low density of sulphate groups per saccharide unit. Complexes of Aubygum x2 (a mixture of kappa and lambda carrageenans) with doxycyclin, emepronium and propranolol were disclosed. The doxycyclin complex is insoluble in water, but in the gastric juice doxycyclin is released at the same rate as from the soluble doxycyclin hyclate. The drug release profile however is strongly pH dependent.

The sulfosalicylate of doxycyclin is known from GB-A-1305860 (Alfa Farmaceutici). This salt is sparing soluble in water and is used in the doxycyclin manufacturing process. The sulfo-salicylic acid salt however has no clinical use, as sulfosalicyclic acid is not accepted for medicinal use.

It has now surprisingly been shown that tetracyclins form sparingly soluble salts with sugar acids, salts which can be used for the controlled release of the tetracyclin antibiotic within the gastrointestinal tract.

The salts with SOS are crystalline and particularly preferred.

The drug:sugar acid salts of the invention may be described by the general formula

(D)ₐ(SA)_{b}(CA)_{c}

where D, SA and CA respectively represent the drug molecule (or a cation thereof), the sugar acid (or an anion thereof) and a physiologically tolerable counterion, a and b are positive numbers (not necessarily integers), and c is zero or a positive number.

Thus, by way of example, the tetracyclin:SOS salts can be described by the following formula:

[sucrose-octa-O-sulphate ⁸⁻] - [TC H⁺]₈, x H₂O]

wherein TC is a tetracyclin molecule, and x is a number of from 0 to 20 indicating the amount of water which forms a hydrate with the new salt or which is physically bound to the new salt.

A further exemplary group of the drug compounds which can be presented according to the invention as their sugar acid salts are the amino glycosides. Examples of suitable aminoglycosides include amikacin, apramycin, arbekacin, bambermycins, butirosin, dibekacin, dihydrostreptomycin, fortimicin, gentamicin, isepamicin, kanamycin, micronimicin, neomycin, netilmicin, paromycin, ribostamycin, sisomicin, streptomycin and tobramycin. Amikacin, gentamicin, kanamycin, neomycin, streptomycin and tobramycin are particularly preferred.

The aminoglycoside:sugar acid salts are particularly suitable for use in the treatment of ulcers, especially stomach or duodenal ulcers and particularly ulceration associated with Helicobacter pylori.

Helicobacter pylori (previously known as Campylobacter pylori) is a helical Gram-negative organism which is present in the stomach mucosa. Many recent tests have shown a clear correlation between the presence of H. pylori in the stomach mucosa and histologically demonstrated gastritis. This seems to indicate that this organism is wholly or partially responsible for the development of gastritis with ensuing ulcerations (see Scand. J. Gastroenterol. (1988) 23 suppl. 142, pages 93-100).

H. pylori is sensitive to a number of known antimicrobial substances in vitro. Furthermore, several publications disclose that the treatment of gastritis with antimicrobial agents, such as β-lactams (e.g. amoxicillin) or bismuth salts can result in the eradication of H pylori in vivo (see Antimicrobial Agents and Chemother. 1993, pages 1184-86).

The traditional treatment of ulceration in the human stomach or the duodenum involves administering acid neutralising agents or anti-histamines of the H2-inhibitor type (e.g. ranitidine, cimetidine, etc.) which reduce the production of acid, and acid pump-inhibitors, such as omeprazole. This treatment as such is efficient, but it has a short-term effect only as, in almost every case, there is a relapse due to H. pylori infection still being present.

Today the optimum treatment of ulceration caused by H. pylori involves combined administration of bismuth subcitrate, amoxicillin and metronidazole. This treatment cures 60-90% of the patients (see Ann. Rev. Med. 1992 (43) page 142).

However, there are certain side effects associated with this therapy: bismuth subcitrate may cause constipation and in large doses it may be neurotoxic; and amoxicillin and metronidazole are systemically acting antibiotics which may cause development of allergy or resistance and influence the microflora in the colon.

Therefore, there is a continuing need for a preparation for local treatment of H. pylori infection in the stomach and the duodenum.

Amino glycosides are a group of antibiotics which have a good in vitro effect on H. pylori. The following MIC-values are known from the literature:

**TABLE 1**

| | Average MIC 90 range |
|---|---|
| AMIKACIN | 0.5 |
| GENTAMYCIN | 0.04 - 1 |
| KANAMYCIN | 0.04 - 2 |
| STREPTOMYCIN | 0.04 - 1.28 |
| TOBRAMYCIN | 0.04 - 0.64 |

(see Antimicrobial Agents and Chemother., 1986, pp. 510-511, J. Antimicrobial Chemother. 1986,'17, pp. 309-314, and Scand. J. Gastroenterol., 1988, 23 (suppl. 142), pp. 93-100).

Thus far however amino glycosides have not been found to be suitable for use in the treatment of H. pylori infections because they are not absorbed following peroral administration of therapeutic doses. When readily soluble salts of amino glycosides are administered orally, they do not influence the stomach and duodenum mucosae due to their poor tissue penetration and thus are not capable of eradicating H. pylori. Instead they will have a considerable influence on the colon microflora and may cause diarrhoea. Amino glycosides could be administered parenterally in ulcer treatment but this is impractical as they have known oto- and nefro-toxic properties.

Amino glycosides belong to the group of sugars having a number of amino groups, preferably 4,5 or 6. They are obtained by fermentation of various Streptomyces- or Micromonospora-species. Due to the many hydrophilic hydroxy or amino groups in the amino glycosides they and the normally used pharmaceutical salts thereof are readily soluble in water.

As the amino glycosides and the sugar acids are generally polybasic it is not immediately predictable that they should together form low water solubility salts.

By way of example the aminoglycoside SOS salts can be represented by the formula

([sucrose-octa-O-sulfonic acid⁸⁻] - [R-(NH₃ ⁺)ₓ]_{y}-M_{z} ⁿ⁺) (I)

where R(NH₂)ₓ is the aminoglycoside (preferably a mono-, di- or trisaccharide), Mⁿ⁺ is a pharmaceutically acceptable cation, preferably alkali metal, alkaline earth metal, aluminium or ammonium ions,
x is an integer having a value of from 1 to 6,
n is an integer having a value of from 1 to 3,
z is zero or a positive number having a value of up to 4,
y is a positive number having a value such that the product of y and x is from 4 to 8, and the sum of the product of x and y and the product of n and z is 8.

It has been found that it is possible to form well defined compounds between sucrose-octa-O-sulfonic acid and an amino glycoside and then to produce mixed salts by introducing cations. The following pharmaceutically acceptable cations are preferred: alkali metal ions, such as Na⁺ and K⁺; ammonium; alkaline earth metal ions, such as Ca²⁺ and Mg²⁺; and aluminium.

The novel drug:sugar acid salts according to the invention preferably have comparatively low solubility in water, such that when stirred into water they form oily or sticky gels with a high degree of affinity to the inside surface of the container. They will preferably also have this affinity for biological surfaces such as the mucosae of the stomach or the duodenum.

Furthermore, the novel salts will preferably release the drug compound in aqueous solutions at a low pH. Therefore, the aminoglycoside salts for example will be suitable for the treatment of ulceration in the stomach and the duodenum caused by H. pylori.

By peroral administration in a suitable pharmaceutical formulation, the novel aminoglycoside salts produce a gel which covers the mucosae of the stomach and/or the duodenum when the stomach pH is neutral or slightly below neutral.

Upon consumption of food, the stomach will secrete hydrochloric acid. H. pylori is sensitive to acid, such as gastric acid, but it has developed a protective measure. This protective measure consists of H. pylori producing an enzyme, urease, which splits urea into ammonia and CO₂. The ammonia thus formed neutralizes the gastric acid.

When the areas of the stomach which are infected with H. pylori come into contact with the aminoglycoside salts according to the invention, the ammonia formed causes release of the amino glycoside which subsequently kills the H. pylori.

If insufficient ammonia to neutralize the gastric acid is formed, the gastric acid will itself release the amino glycoside from the novel salts.

Other examples of amine group containing antibiotics which can be used to form poorly water soluble drug:sugar acid salts according to the invention include polypeptides (such as bacitracin and polymyxin), glycopeptides (such as vancomycin), and macrolides (such as erythromycin and oleandomycin), streptomycins and penicillins.

These poorly water soluble salts can again advantageously be used to achieve controlled release of the antibacterial agent within the gastrointestinal tract, e.g. following peroral administration.

The drugs usable in this fashion are not restricted to the antibiotics and indeed poorly water soluble salts can be produced with a wide range of acid salt forming drug compounds, for example neuroleptics, antidepressants, e.g. tricyclic antidepressants (such as imipramine), the structurally related tricyclic muscle relaxants (such as cyclobenzaprine), anticholinergics, antihistamines, antianorexics, cardioprotective azepine derivatives (such as benzothiazepinones like diltiazem), and alkaloids (such as the antitussive agent noscapine).

Further drug categories which may be considered include antivirals (such as acyclovir, idoxuridine and tromantadine), antimycotics (such as miconazole, ketoconazole, fluconazole, itaconazole, econazole, terconazole, and polyenes such as amphotericin B or nystatin), anti-amoebics (such as metronidazole and tinidazole), antihistamines (such as diphenylhydramine, chlorpromazine, pyrilamine and phenyltoloxamine), calcium agonists (such as verapamil and nifedipine), anxiolytics, sedatives and hypnotics (such as benzodiazepines, diazepam, nitrazepam, flurazepam, estazolam, flunitrazepam, triazolam, alprazolam, midazolam, temazepam, lormetazepam, brotizolam, clobazam, clonazepam, lorazepam and oxazepam), anti-migraine agents (such as sumatriptan), anti-motion sickness agents (such as cinnarizine), anti-emetics (such as ondansetron, tropisetron and granisetrone), adrenergics (such as amphetamine), antispasmodics (such as aminopentamide, metixene and dicyclomine), ataractics (such as benactyzine), antihypertensives (such as hexamethonium and pentamethonium), analgesics and alkaloids (such as 2,6-diamino-3-phenyl-azopyridine, morphine, papaverine and ethaverine), antitussives (such as dihydrocodeine, phenylpropanolamine, guaiacol, cloperastine and dextromorphen), bronchodilators (such as dimethylephedrine), antipsychotics (such as imipramine), coronary dilators (such as etafenone), antiarrhythmics (such as procainamide), hypotensives (such as hydralazine and clonidine) and peripheral vasoconstrictors (such as tolazoline).

Further examples of amine containing drug compounds include acetophenazine, amitriptyline, brompheniramine, carbinoxamine, chlorcyclizine, cyclizine, desipramine, dexbrompheniramine, dexchlorpheniramine, ergotamine, nortriptyline, quinidine, benztropine, flunarizine, fluphenazine, hydroxychloroquine, hydroxyzine, meclizine, mesoridazine, methdilazine, methysergide, pheniramine, pyrilamine, tripelennamine, triprolidine, promazine and quinidine.

The new drug:sugar acid salts may be prepared particularly simply by containing a water soluble form of the drug, either the free base or a water soluble salt thereof (e.g. a hydrochloride), with a water soluble form of the sugar acid, optionally in the presence of further counterions which it is desired to precipitate as part of a drug:sugar acid mixed salt. Contacting will generally be effected in a solvent or solvent mixture, optionally a water-miscible solvent system in which drug, sugar acid and salt are all soluble in which case salt precipitation may be effected by addition of water.

Various basic drug compounds do not form water-insoluble sugar acid salts; however it is of course readily determined by simple experimentation whether or not a poorly soluble salt forms.

In general, if a 100mM solution of the drug hydrochloride in ion-exchanged water is mixed with an equinormal amount of sugar acid (i.e. an acid group:drug molecule ratio of 1:1) also in solution in ion-exchanged water, e.g. in a volume about 3/4 of that of the drug solution, a poorly soluble drug:sugar acid salt would precipitate.

The novel salts are particularly advantageously produced by allowing an aqueous solution of the drug base to react with an aqueous solution of sugar acid by titration to obtain the desired stoichiometric ratio.

If it is desired to produce salts in a 1:1 ratio it may be necessary to neutralise excess acid groups in the sugar acid with suitable cations, such as Na⁺, Ca⁺, Mg²⁺ or Al³⁺. In many instances, the salts thus formed will crystallize spontaneously. If this is not the case the salt may be obtained by evaporation, optionally to dryness, or by freeze drying, or by addition of a solvent which is miscible with water, such as methanol or ethanol.

By way of example, a soluble aminoglycoside (e.g. kanamycin A) may be dissolved in water and a solution of SOS may be added thereto. The drug:sugar acid salt separates out as a syrup which can be crystallized from ethanol.

As a further example, to a solution of a tetracyclin (e.g. doxycyclin) in hydrochloric acid there may be added an aqueous solution of SOS-sodium salt. The drug:sugar acid salt precipitates out.

Where inclusion of aluminium as a counterion is desired, the initial aluminium containing aqueous solution should be at a low pH, e.g. about 4, and the precipitation is accomplished by raising the pH, e.g. to 5.5 to 6. The aluminium salt solution may be brought into contact with the drug or drug salt before the sugar acid is added if the drug or drug salt will remain stable in solution in that environment. Otherwise all three components may be brought together simultaneously, optionally with subsequent addition of a base to raise the pH to complete precipitation.

A further option is to effect ion exchange between aluminium, in an insoluble aluminium sugar acid salt, and the drug cations. This requires careful pH control, e.g. to about pH 4, to ensure the exchange reaction occurs.

Thus it is possible to use sucralfate as a starting material. In this case, by way of example the chosen drug is dissolved in water and the pH is adjusted to 4.0. To this solution, an equivalent amount of sucralfate is added with vigorous stirring. An ion exchange reaction takes place whereby the drug forms ion bonds with one or more of the sulphonic acid groups in sucrose-octa-O-sulphonic acid and a corresponding amount of aluminium passes into the aqueous phase. When the reaction is completed the novel salts are filtered off and washed with water.

This reaction will only take place in a very narrow pH range, i.e. around pH 4. If pH is higher than 4, the Al-ion is not dissolved and, therefore, it cannot be separated from the reaction product by filtration. If pH is lower than 4, a gel formation takes place which also makes a separation impossible. Furthermore, at too low a pH, the equilibrium may move in such a manner that the drug will remain dissolved.

Thus viewed from a further aspect the invention provides a process for the preparation of poorly water-soluble sugar acid salts of bioactive organic compounds, said process comprising reacting said compound or a salt thereof with said acid or a salt thereof in a solvent system and separating said salt from said system, e.g. by filtration.

The salts can be obtained directly, on via recrystallation, as surprisingly poorly-soluble, well crystallized material.

Because of the new salts' low solubility in water, the main part of the active drug will be bound to the sugar acid moiety and thus remain biologically inactive during the passage of the oesophagus. This greatly reduces the risk of unfavourable interaction with the mucosa in the oesophagus.

When pharmaceutical formulations are made which contain the new salt for oral use, or peroral administration, the salt can be mixed with a solid pulverised carrier, such as: calcium carbonate, calcium phosphate, calcium crospovidone sulphate, microcrystalline cellulose, cellulose dextrates, dextrin, dextrose excipient, fructose, lactose, mannitol, sorbitol, starch povidone, pregelatinized starch, sucrose, compressible sugar or confectioner's sugar; and can also contain lubricants, such as: calcium stearate, magnesium stearate, polyethylene glycol, stearic acid, talc, or zinc stearate. A tabletting mixture may be prepared and mixed with other subsidiary materials before being compressing it into tablets. If coated tablets are desired, then the core - made as outlined above - might be coated with: sodium carboxymethylcellulose, cellulose acetate, cellulose acetate phthalate, ethylcellulose, gelatin, pharmaceutical glaze, hydroxypropyl cellulose, hydroxypropyl methylcellulose, hydroxypropyl methylcellulose phthalate, methylacrylic acid copolymer, methylcellulose, polyethylene glycol, polyvinyl acetate phthalate, shellac, sucrose, titanium dioxide, carnauba wax, microcrystalline wax or zein which is first dissolved in water an organic solvent or a mixture of solvents. Colouring agents can be added to this dissolution, so that tablets with different strengths can be identified.

Powders and granulates can be made, which contain the new salt alone or mixed with a solid pulverulent carrier, such as: calcium carbonate, calcium phosphate, calcium crospovidone sulphate, microcrystalline cellulose, cellulose, dextran, dextrin, dextrose excipient, fructose, lactose, mannitol, sorbitol, starch, povidone, pregelatinized starch, sucrose, compressible sugar, or confectioner's sugar; and can also contain lubricants, such as: calcium stearate, magnesium stearate, polyethylene glycol, stearic acid, talc, or zinc stearate. The remainder can be comprised of, for example, sweetening agents, such as sugar, aspartame, dextran, dextrose, fructose, mannitol, saccharin, calcium saccharin, sodium saccharin, sorbitol or sucrose.

The powders or granulates may be dissolved and/or dispersed before use. They may also be mixed with a foodstuff before administration.

Soft gelatin capsules can be made, which contain a mixture of the salt and/or vegetable oil. Hard gelatin capsules can contain granulates of the salt in combination with a solid pulverulent carrier such as: calcium carbonate, calcium phosphate, calcium crospovidone sulphate, microcrystalline cellulose, cellulose, dextran, dextrin, dextrose excipient, fructose, lactose, mannitol, sorbitol, starch, povidone, pregalatinized starch, sucrose, compressible sugar, or confectioner's sugar, or the new salt alone; and can also contain lubricants, such as: calcium stearate, magnesium stearate, polyethylene glycol, stearic acid, talc, or zinc stearate.

Liquid formulations which may be used for oral use, or peroral administration, may be made in the form of syrups, suspensions, emulsions, or mixtures which can contain up to approximately 20% of the new salt. The remainder can be comprised of, for example, sweetening agents, such as sugar, aspartame, dextran, dextrose, fructose, mannitol, saccharin, calcium saccharin, sodium saccharin, sorbitol or sucrose and a mixture of diluents, like ethanol, water, glycerol and propylene glycol.

The dose in which the new salt is administered is dependant on different factors, like for example the nature of the drug compound and individual needs and administration preferences of each patient. The dosages will generally be on the same level as normally used for the drug compound.

By way of example, dosage units containing 1 to 1000mg, conveniently 50 to 200mg, and preferably about 100mg of the drug compound per unit will be preferred.

For doxycyclin salts, for example, dosages of 100 to 400mg/day of the drug compound will normally be required. As a further example, for the amine glycosides, it will be preferred to administer the novel salts in tablets or capsules which disintegrate in the stomach or duodenum and which each contain from 50 to 250mg, preferably about 100mg, of the amino glycoside together with conventional adjuvants and/or carriers.

The dissolution properties of the new salts according to the invention can easily be demonstrated by the method described in the Examples below.

By modifying the composition of the new salts according to the invention, it is possible to change the dissolution properties in order to optimize the compositions with regard to the dissolution profile desired.

The new salts according to the present invention can also be used locally on the skin, or mucous membranes formulated as creams, lotions, ointments, or gels. As an example, doxycylin sucrose octasulphate is well suited for insertion in or around the periodontal pocket of an individual suffering from periodontitis. In this case, the vehicle described in US-A-5143934 (Dumex) is especially advantageous to use as a carrier.

In the accompanying drawings, Figure 1 is a plot of dissolution rate for doxycyclin.SOS salt according to the invention compared with the commercial product Vibramycin, Figure 2 is a schematic representation of apparatus used for the determination of drug retention on gastrointestinal mucous membrane, and Figure 3 is a schematic representation of apparatus which may be used to evaluate bioadhesion by tensiometry.

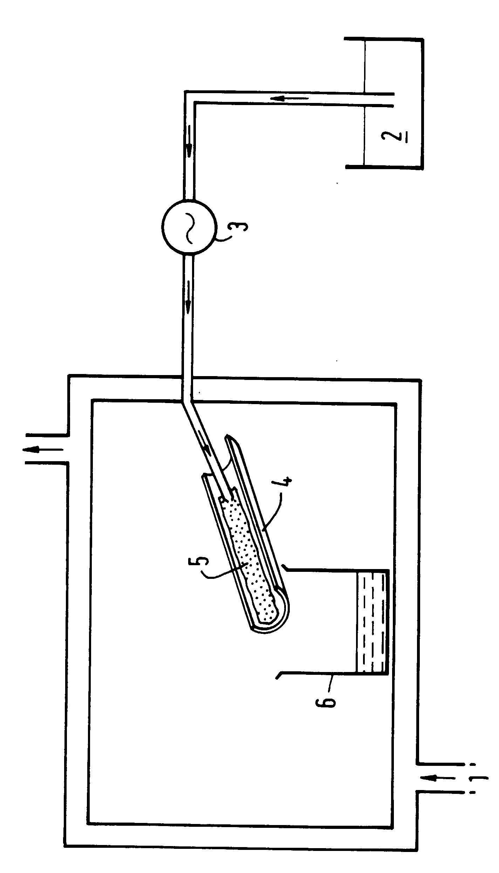

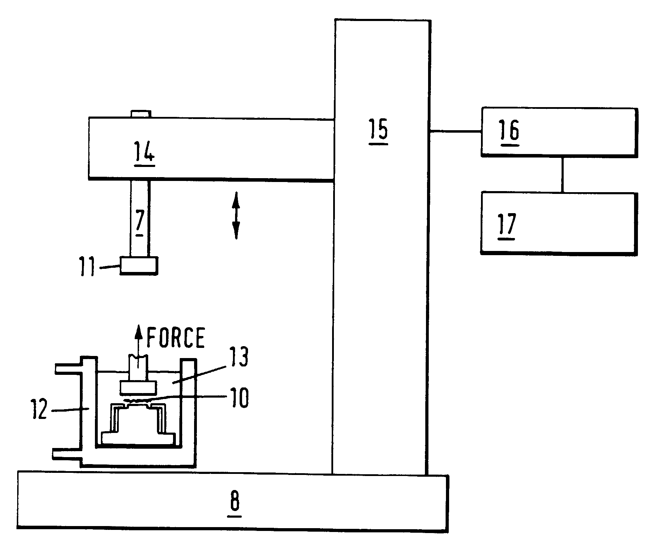

The bioadhesion properties of the drug:sugar acid salts of the invention may be tested in vitro on the test systems described below with reference to Figures 2 and 3 of the accompanying drawings.

### 1. In vitro test system for bioadhesion by means of rabbit jejunum membranes

The bioadhesive test system described in the following is a modified system of a method described by Ranga Rao and Buri, 1989.

Male albino rabbits (3-4 kg) (New Zealand white rabbit SSC:CPH) were fasted for 20 hours before they were killed by means of a pentobarbital sodium injection. The intestines of the rabbits were dissected, and placed in an isotonic 0.9% sodium chloride solution at ambient temperature (about 18°C). Within 30 minutes the jejunums were cut and washed with 0.9% sodium chloride solution. The lumens were gently rinsed with the saline until the intestines were clean. The jejunums were cut into pieces of 8-9 cm and immediately frozen (-20°C). The intestines were stored up to 3 months before use. Before testing, the segment of jejunum was gently thawed out.

The segment of jejunum was cut longitudinally. It was placed on a stainless steel support (a tube of 2 cm in diameter and cut longitudinally at its centre) with the mucosa layer facing up, spread and held in position on the support by the adhesive effect of the jejunum itself. The support with the jejunum was placed at an angle of -7 to -21° in a cylindrical cell thermostated at 37°C. A schematic description of the cell is shown in Fig. 2. The relative humidity in the thermostatic cell was kept at 100%. The intestines were then flushed with isotonic 0.02 M phosphate buffer solution (pH 6.5, 37°C) for 5 minutes at a flow rate of 10 ml min⁻¹, using a peristaltic pump, to equilibrate the intestine with the buffer and to rinse off loose mucosa. An accurately weighed amount of the sample to be tested for bioadhesiveness (about 50-150 mg) was placed evenly on the mucosa of the jejunum (0.8 x 6 cm). About 1 ml of the buffer solution was carefully dropped evenly onto the sample to ensure hydration. Immediately after, the segments were left for 10 minutes in the cell allowing the sample to interact with the glycoproteins of the jejunum and to prevent drying of the mucus. After 10 minutes the segments were evenly flushed with the isotonic 0.02 M phosphate buffer solution (pH 6.5, 37°C) for 30 minutes at a constant flow rate of 5-15 ml min⁻¹. The tip of the tube carrying the buffer solution was placed 3-4 mm above the jejunum to ensure an even liquid flow over the mucosa. The effluent was collected into a beaker. The amount of bioadhesive component remaining on the jejunum was calculated either by measuring the amount of compound remaining on the jejunum or by measuring the amount in the receiver by means of HPLC. Other tissue than rabbit jejunum may be applied. E.g. buccal mucosa, stomach, intestine obtained from e.g.. pig, rat and rabbit.

### 2. In vitro test system for bioadhesion by means of tensiometry

The test system for bioadhesion described in the following is a modified system of a method described by Tobyn, Johnson and Gibson (in "Use of a TA.XT2 Texture Analyser in Mucoadhesive Research", International LABMATE, 1992, XVII (issue VI), 35-38).

The test system involves measuring the tensile force required to break an adhesive bond formed between a model membrane and a test sample (i.e the sample which is tested for its bioadhesive properties).

The test apparatus employed in the following is a TA.XT2 Texture analyser (Stable Micro System Ltd., Haslemere, UK) (Figure 3). The test enables measuring the strength of adhesive bonding established by contacting a model membrane, i.e. in this case a rabbit intestine segment, and the test sample. An analogous test apparatus may also be employed.

The TA.XT2 Texture analyser apparatus is equipped with an instrument probe 7 (see Figure 3) on a sliding stand 14 which is movable in a vertical direction at a variable rate under the control of a motor and displacement transducer 15 operated by control unit 17 and computer 17. During the so-called withdrawal phase of the testing, the instrument probe is moved upwards with a constant rate until detachment occurs (see below). Furthermore, the apparatus is equipped with a stationary place 8 on which a first holder 9 is placed. Before and during a test run, a model membrane 10 is fixed on this holder, e.g. by means of a cap or double adhesive tape or glue. The holder is constructed so that a well-defined area of the model membrane (about 0.5-9 cm²) is used in the test runs. The accurate size of the exposed area is used in the calculation of the adhesive strength (see below).

As mentioned above, the test involves employment of a model membrane, primarily of animal origin. The membrane could be e.g. rabbit, rat or pig gastric mucosa; a segment of rabbit, rat or pig intestines, e.g. a segment of rabbit jejunum; or a segment of rabbit, rat or pig intestines from which the mucosal layer has been removed prior to testing; or skin from an animal (after removal of substantially all subcutaneous fat); or it could be artificial or commercial available mucin.

In the test described below, a model membrane of a segment of rabbit intestines, i.e. a segment of rabbit jejunum has been employed. However, it is appreciated that a change of membrane in some case may be advantageous, e.g. if a relatively large surface area of a model membrane is required. If the results obtained by use of another membrane than the rabbit intestine model membrane are used to compare the bioadhesive properties of various substances or combinations, the results of a reference compound could be included. As discussed below testing of a reference sample may also be made as a routine. Polycarbophil and Carbopol 934 have been found suitable as reference compounds.

A test sample (about 50-500 mg) is applied in a uniform layer either
i) on the luminal side of the model membrane 10 placed on the first holder 9, or
ii) directly on the instrument probe 7, if necessary by means of a double adhesive tape or glue applied on the instrument probe before application of the test sample.

In those cases where it is not possible to fix the test sample to the instrument probe, the apparatus may be equipped with a second holder 11 on which another model membrane is fixed. In such cases, the model membranes employed on the two holders are usually of the same type (e.g. segments of rabbit jejunum). It is also possible to fix the other model membrane directly to the instrument probe e.g. by means of a duple adhesive tape or glue.

Test runs are usually performed in an aqueous medium and the temperature is maintained at 37°C by use of a thermostatically controlled heater/stirrer 12. The aqueous medium is contained in a vessel 13. Prior to testing, the model membrane and the test sample is allowed to equilibrate with the aqueous medium for about 5-30 minutes. The aqueous medium is usually added during or after the initial test phase in which the two substrates (i.e. the model membrane and the test sample) are gently contacted with each other by means of lowering the instrument probe. As aqueous medium is used isotonic 0.02 M phosphate buffer solution, pH 6.5 but it may be replaced by other aqueous solutions.

In some cases it is desirable to avoid testing in an aqueous medium (e.g. in those case where the test sample has a relatively low dynamic viscosity); however, in order to avoid drying of the model membrane, a solvent evaporation trap may be employed, whereby the temperature (about 37°C) and the humidity (at least about 80%) are controlled during testing. In those cases where the test sample can form a fluid crystalline phase, it may also be necessary to add a sufficient amount of water in order to induce formation of such a fluid crystalline phase.

### Test runs are performed as follows:

The instrument probe (either with or without a second holder 11) is lowered in order to bring the model membrane and the test sample in contact under a constant force (preload of 0.05-2 N). After a time period of about 30 sec-3 minutes of contact time, the withdrawal phase is initiated by applying a vertically acting force by raising the instrument probe at a constant speed of about 0.1-0.2 mm sec⁻¹ until the two substrates (i.e. the model membrane as a first substrate and the test sample as a second substrate) are completely detached. The force required for detachment is recorded. Data is continuously collected and calculations are performed by means of a software program "XTRA Dimension software package" available from Stable Micro Systems, UK. The maximum force of detachment represents the adhesion strength (N cm⁻²) and the area under the force/time (or force/distance) curve is considered as the adhesion work (mJ) .

### Determination of the bioadhesive properties of a test sample

In order to test whether a test sample is bioadhesive, two test runs are performed:
1. A test run without any test sample applied (result: adhesion strength T₀)
2. A test run with the test sample applied (result: adhesion strength T).

In both cases the adhesion strength is calculated and the test sample is considered bioadhesive if T/T₀ x 100% is at least 115%, such as 125%, 135%, 150%, 175%, 200%.

Alternatively, a test run with the test sample is performed and the results are compared with the results of testing known bioadhesive substances such as, e.g. polycarbophil (a strong bioadhesive substance), or chitosan, tragacanth, hydroxypropylmethylcellulose (HPMC), hydroxypropylcellulose (HPC), karaya gum, carboxymethylcellulose (CMC), gelatin, pectin, acacia, PEG 6000, povidone, or DEAE-dextran (less bioadhesive than polycarbophil).

Polycarbophil (Noveon™ AA-1, BF Goodrich, Hounslow, U.K.) is a high molecular weight poly(acrylic acid)copolymer loosely cross-linked with divinyl glycol. On account of its known excellent mucoadhesive properties, this polymer serves as a reference. Before testing in the above-mentioned tensiometric test, a polycarbophil gel is prepared by mixing polycarbophil with water (resulting concentration about 10-20 mg ml⁻¹) and the mixture is allowed to hydrate at 37°C for 24 hours. The polymer solution is periodically stirred. The pH is adjusted to 5.1 using either diluted sodium hydroxide or hydrochloric acid. The resulting gel is tested as described above and the result obtained is used as a reference value for excellent bioadhesive substances. Similarly, other substances which are known bioadhesive substances are tested, and by choosing test substances with various degrees of bioadhesiveness so that an evaluation scale can be made, the performance of a test sample with respect to bioadhesiveness can be evaluated. It is contemplated that the following scale is applicable (force resolution 0.1 gm):

| Bioadhesive properties | Adhesion force (mN cm⁻²) |
|---|---|
| none | less than 0.1 mN cm⁻² |
| poor | about 0.1 - about 1 mN cm⁻² |
| moderate | about 1 - about 4 mN cm⁻² |
| good | about 4 - about 10 mN cm⁻² |
| excellent | more than 10 mN cm⁻² |

In some cases, e.g. in the case of tablets with excellent bioadhesive properties, an adhesion force may be as high as 25 about 700 mN cm⁻² or even higher.

### 3. In vitro test system for bioadhesion by means of tensiometry - determination of duration of adhesion

The test system described in the following is a modified system of a method described by Smart (Int. J Pharm. 73:69-74(1991)).

The apparatus and conditions employed in this test are the same as described in the test denoted 2 above.

The duration of adhesion is evaluated by applying a constant tensile force between about 0.25-2 N cm⁻², such as 1 N cm⁻², to the adhesion bond after the initial contact time (see above: 0.5-3 minutes) and leaving for up to 8 hours or until the bond fractures. Every hour the tensile force acting on the adhesive joint is noted and if necessary adjusted to the initial value (eg 1 N cm⁻²). After 8 hours the force required to break the adhesive bond is evaluated.

All publications referred to hereinbefore are incorporated herein by reference.

The invention will now be described by reference to the following non-limiting Examples.

### EXAMPLE 1

### Sucrose-octa-O-sulphonic acid

60g (50mmol) of sucrose-octa-O-sulphonic acid sodium salt (produced in accordance with J. Chem. Soc. Faraday Trans., 1981, 77, pages 629-639) are dissolved in 200ml of water and cation exchanged on Amberlite® IR (H⁺). The combined eluates are diluted to 1 litre corresponding to an 0.05 M solution.

### EXAMPLE 2

### Doxycyclin sucrose-octa-O-sulphonic acid salt

18.5g (40mmol) doxycyclin monohydrate is dissolved in 400ml 0.1 M HCl and by addition of 6.5g (5mmol), sucrose-octa-O-sulphonic acid-Na₈, 8 aq., dissolved in 300ml water, precipitation of doxycyclin sucrose octasulphate takes place. The reaction mixture is stirred for 60min at 25°C, filtered, washed with 3x50ml water by dispersion and refiltration and vacuum dried at 1 Torr/25°C/sicapent®/20h.
Yield: 23.5g.
MP.:150°C (decomp.).

Stoichiometric proportions: in the batches produced, the content of water was found to be about 10%. The content of doxycyclin was found to be about 77%, calculated with reference to the dried substance. This content of doxycylin indicates that 8 moles of doxycyclin complex with 1 mole of sucrose-octa-O-sulphonic acid.

### EXAMPLE 3

### Doxycyclin SOS salt

20.5g doxycylin hyclate (40mmol) is dissolved in 400ml water and with vigorous stirring, 100ml of the acid solution of Example 1 is added. After 30min, the pH is adjusted to 3.5 with 0.1 M NaOH solution. After another 30min of stirring, the precipitate is filtered and dried as in Example 2.

### EXAMPLE 4

### Doxycyclin SOS salt

18.5g (40mmol) doxycyclin monohydrate is dissolved in 400ml 0.1 M HCl and by titration with 100ml 0.05 M (5mmol) sucrose-octa-O-sulphonic acid, precipitation of doxycyclin sucrose octa sulphate takes place. The reaction mixture is stirred for 60min at 25°C, filtered, washed with 3x50ml water (by dispersion and refiltration) and vacuum dried at 1 Torr/25°C/sicapent®/20h.

### EXAMPLE 5

### Doxycyclin SOS salt

By titration of 6.5g (5mmol) sucrose-octa-O-sulphonic acid Na₈ , 8aq. dissolved in 300ml water, with 18.5g (40mmol) doxycylin monohydrate dissolved in 400ml 0.1 M HCl, precipitation of doxycyclin sucrose octasulphate takes place. The reaction mixture is stirred for 60min at 25°C, filtered, washed with 3x50ml water (by dispersion and refiltration) and vacuum dried at 1 Torr/25°C/sicapent®/20h.

### EXAMPLE 6

### Dissolution of doxycyclin sucrose octasulphate

The paddle method is used in accordance with USP XXII, p. 1579, (apparatus II), with 900ml 0.1 N HCl. One tablet containing 150mg doxycyclin sucrose octasulphate (equal to 105mg doxycyclin) is placed in the dissolution apparatus. Samples are taken after 5, 10, 20, 30 and 60 minutes and diluted.

The samples are analyzed spectrophotometrically at 345.9nm in a Shimadzu 160 A photometer.

The results are compared to samples of Vibramycin® from Pfizer, which contains doxycyclin carragenate.

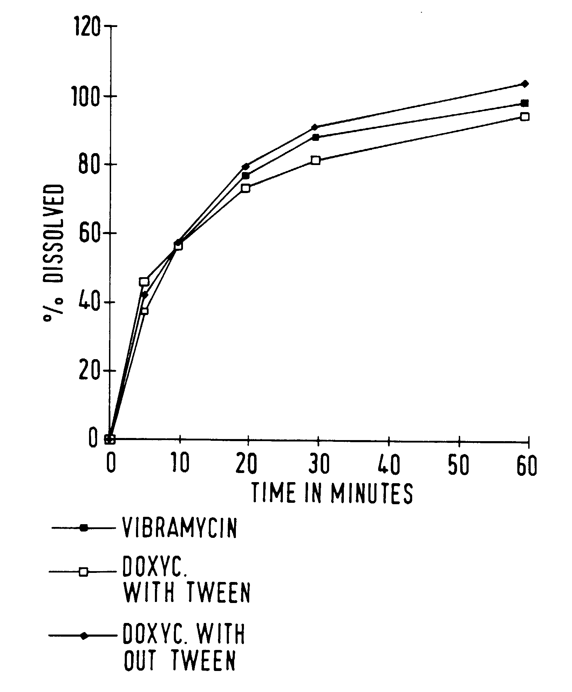

In Figure 1, the plot of release versus time is illustrated graphically. This shows that the delayed release characteristics are similar despite the major differences in the counterion.

In Figure 1, data points for Vibramycin are indicted by solid squres (■), for Doxycyclin without Tween by hollow squares (□) and for Doxycyclin with Tween by solid diamonds (◆). The data point values are set out in Table 2 below:

**TABLE 2**

| Comparison of Vibramycin and Doxycyclin-SOS Tablets 100 MG | | | |
|---|---|---|---|
| Time | Vibramycin | Doxyc. with tween | Doxyc. without Tween |
| 0 | 0 | 0 | |
| 5 | 37.45 | 45.89 | 42.02 |
| 10 | 56.06 | 56.12 | 57.22 |
| 20 | 76.75 | 73.31 | 79.54 |
| 30 | 88.08 | 81.42 | 91.06 |
| 60 | 98.67 | 94.76 | 104.29 |

### EXAMPLE 7

The reaction of a drug:sugar acid salt of the invention with water is a swelling reaction whereby a sticky substance or syrup is formed. The absorption of water occurs to a certain point where the particles have become an oily, viscous fluid which has comparatively low solubility in water. When a drop of this fluid is applied to smooth skin, a membrane is formed which has low solubility in water and which may only be removed by intensive scrubbing with water. By rinsing with ion-containing water, the membrane is gradually dissolved until it disappears completely.

### EXAMPLE 8

### Test for bioadhesion on mucosal surfaces

The bioadhesion of the novel salts according to the invention is demonstrated in the test system shown in Figure 2 wherein (1) is thermostatic water flow at 40°C, (2) is a reservoir containing the washing solution at 37°C, (3) is a peristaltic pump, (4) is a stainless steel support, (5) is a model membrane, and (6) is a receiver for collecting the washings.

A segment of jejunum from rabbit is placed on a support (4) with the mucosa facing upwards, spread and held in position on the support by the adhesive effect of the jejunum itself. Support and jejunum are placed at an angle of -7°C in a cylindrical cell thermostated at 37°C. The sample to be tested is placed on the jejunum and lml buffer is dropped thereon. The buffer used is either a 0.01 M HCl solution, pH 2 (buffer a) or borate buffer solution 0.05 M, pH 7.4 (buffer b). The segments are left for 10 minutes in the cell to allow the test substance to interact with the glycoproteins of the mucosa. After 10 minutes the jejunum is flushed with either buffer a or b for 30 minutes.

The buffer is collected in a receiver (6). The amount of test substance remaining on the jejunum is calculated by measuring the amount in the receiver by means of HPLC.

### EXAMPLE 9

### Diltiazem Salt of Sucrose-octa-O-sulphonic Acid

16.7109 g (37.0546 mmol) Diltiazem·HCl is dissolved in 400 ml ion-exchanged water (pH 4.42) and by addition of 6.5281 g (5.6344 mmol) sucrose-octa-O-sulphonic acid-Na₈, 8 aq., dissolved in 200 ml ion-exchanged water, precipitation takes place. The reaction mixture is filtered and vacuum dried at lTorr/25°C/20h/Sicapent®.
Yield: 19.1166 g
DSC showed decomposition from 170°C.
Water content: 3.9%

### EXAMPLE 10

### Cyclobenzaprine Salt of Sucrose-octa-O-sulphonic Acid

Analogously to Example 9, using 12.4763 g (40.0087 mmol) Cyclobenzaprine·HCl in 400 ml ion-exchanged water (pH 4.15) and 5.8159 g (5.0198 mmol) sucrose-octa-O-sulphonic acid-Na₈, 8 aq., in 300 ml ion-exchanged water.
Yield: 4.5709 g
DSC showed decomposition from 200°C.

### EXAMPLE 11

### Noscapine Salt of Sucrose-octa-O-sulphonic Acid

Analogously to Example 9, using 17.9965 g (40.0020 mmol) Noscapine.HCl in 400 ml ion-exchanged water (pH 3.86) and 5.7910 g (4.9983 mmol) sucrose-octa-O-sulphonic acid-Na₈, 8 aq., in 300 ml ion-exchanged water.
Yield: 17.0296 g
DSC showed decomposition from 200°C.

### EXAMPLE 12

### Amitriptyline salt of Sucrose-octa-O-Sulphonic Acid

Analogously to Example 9, using 12.5245 g (39.9009 mmol) Amitriptyline·HCl in 400 ml ion-exchanged water (pH 3.73) and 5.7715 g (4.9814 mmol) sucrose-octa-O-sulphonic acid-Na₈, 8 aq., in 300 ml ion-exchanged water.
Yield: 2.1166 g

### EXAMPLE 13

### Chlordiazepoxide salt of Sucrose-octa-O-Sulphonic Acid

Analogously to Example 9, using 13.4300 g (39.9370 mmol) Chlordiazepoxid·HCl in 400 ml ion-exchanged water (pH 3.08) and 5.7774 g (4.9865 mmol) sucrose-octa-O-sulphonic acid-Na₈, 8 aq., in 300 ml ion-exchanged water.
Yield: 21.8657 g

### EXAMPLE 14

### Erythromycin Salt of Sucrose-octa-O-sulphonic Acid

9.9861 g (6.8941 mmol) Erythromycin Lactobionate is dissolved in 400 ml ion-exchanged water (pH 6.74). The solution is adjusted to pH 4.00 with 1M HCl (pH 4.33). A solution of 1.0021 g (0.8649 mmol) sucrose-octa-O-sulphonic acid-Na₈, 8 aq., in 300 ml ion-exchanged water is added, which results in precipitation. The reaction mixture is filtered and vacuum dried at lTorr/25°C/20h/Sicapent®.
Yield: 3.8896 g
DSC showed decomposition from 200°C.

### EXAMPLE 15

### Imipramine Salt of Sucrose-octa-O-sulphonic Acid

Analogously to Example 14, using 3.9789 g (12.5573 mmol) Imipramine·HCl in 200 ml ion-exchanged water (pH 5.46) and 1.8809 g (1.6234 mmol) sucrose-octa-O-sulphonic acid-Na₈, 8 aq., in 100 ml ion-exchanged water.
Yield: 0.7618 g

### EXAMPLE 16

### Nortriptyline Salt of Sucrose-octa-O-sulphonic Acid

Analogously to Example 14, using 0.7358 g (2.4540 mmol) Nortriptyline·HCl in 100 ml ion-exchanged water (pH 6.22) and 0.3647 g (0.3148 mmol) sucrose-octa-O-sulphonic acid-Na₈, 8 aq., in 100 ml ion-exchanged water.
Yield: 0.7844 g
DSC showed decomposition from 190°C.

### EXAMPLE 17

### Quinidine Salt of Sucrose-octa-O-sulphonic Acid

Analogously to Example 14, using 3.8360 g (5.1357 mmol) Quinidine Sulphate in 400 ml ion-exchanged water (pH 6.29) and 0.7505 g (0.6478 mmol) sucrose-octa-O-sulphonic acid-Na₈, 8 aq., in 100 ml ion-exchanged water.
Yield: 1.4638 g
DSC showed decomposition from 175°C.

### EXAMPLE 18

### Benztropine Salt of Sucrose-octa-O-sulphonic Acid

Analogously to Example 14, using 4.0353 g (9.5739 mmol) Benztropine Mesylate in 300 ml ion-exchanged water (pH 6.23) and 1.4011 g (1.2093 mmol) sucrose-octa-O-sulphonic acid-Na₈, 8 aq., in 150 ml ion-exchanged water.
Yield: 3.0020 g

### EXAMPLE 19

### Verapamil Salt of Sucrose-octa-O-Sulphonic Acid

Analogously to Example 14, using 0.7512 g (1.5298 mmol) Verapamil·HCl in 200 ml ion-exchanged water (pH 6.17) and 0.2226 g (0.1921 mmol) sucrose-octa-O-sulphonic acid-Na₈, 8 aq., in 100 ml ion-exchanged water.
Yield: 0.2791 g

### EXAMPLE 20

### Chlorpromazine salt of Sucrose-octa-O-Sulphonic Acid

Analogously to Example 14, using 11.8682 g (33.3949 mmol) Chlorpromazine·HCl in 400 ml ion-exchanged water (pH 5.10) and 4.9071 g (4.2354 mmol) sucrose-octa-O-sulphonic acid-Na₈, 8 aq., in 300 ml ion-exchanged water.
Yield: 8.2810 g

### EXAMPLE 21

### Diphenhydramine salt of Sucrose-octa-O-Sulphonic Acid

Analogously to Example 14, using 5.9032 g (20.2268 mmol) Diphenhydramine·HCl in 200 ml ion-exchanged water (pH 5.42) and 2,9330 g (2,5315 mmol) sucrose-octa-O-sulphonic acid-Na₈, 8 aq., in 150 ml ion-exchanged water.

### EXAMPLE 22

### Tobramycin salt of Sucrose-octa-O-Sulphonic Acid

Analogously to Example 14, using 18.6368 g (39.8614 mmol) Tobramycin in 400 ml ion-exchanged water (pH 10.36) and 5,7753 g (4,9847 mmol) sucrose-octa-O-sulphonic acid-Na8, 8 aq., in 300 ml ion-exchanged water.
Yield: 9.5791 g

### EXAMPLE 23

### Bacitracin Salt of Sucrose-octa-O-sulphonic Acid

11.8176 g (8.3074 mmol) Bacitracin is dissolved in 400 ml 0.1M HCl (pH 2.16) and by addition of 5.7098 g (4.9282 mmol) sucrose-octa-O-sulphonic acid-Na₈, 8 aq., dissolved in 300 ml ion-exchanged water, precipitation takes place. The reaction mixture is filtered and vacuum dried at 1Torr/25°C/20h/Sicapent®.
Yield: 7.5705 g
DSC showed decomposition from 150°C.

### EXAMPLE 24

### Polymyxin Salt of Sucrose-octa-O-sulphonic Acid

Analogously to Example 23, using 10.0453 g (8.3711 mmol) Polymyxin in 400 ml 0.1M HCl (pH 1.60) and 1.2384 g (1.0689 mmol) sucrose-octa-O-sulphonic acid-Na₈, 8 aq., in 200 ml ion-exchanged water.
Yield: 4.3644 g
DSC showed decomposition from 160°C.

### EXAMPLE 25

### Vancomycin Salt of Sucrose-octa-O-sulphonic Acid

Analogously to Example 23, using 7.6646 g (5.2888 mmol) Vancomycin in 200 ml 0.1M HCl (pH 1.58) and 0.8011 g (0.6914 mmol) sucrose-octa-O-sulphonic acid-Na₈, 8 aq., in 100 ml ion-exchanged water.
Yield: 2.8677 g
DSC showed decomposition from 135°C.

### EXAMPLE 26

### Flunarizine Salt of Sucrose-octa-O-sulphonic Acid

Analogously to Example 23, using 0.7635 g (1.5992 mmol) Flunarizine·2HCl in 200 ml 0,1M HCl (pH 1.31) and 0.2414 g (0.2084 mmol) sucrose-octa-O-sulphonic acid-Na₈, 8 aq., in 100 ml ion-exchanged water.
Yield: 0.6669 g

### EXAMPLE 27

### Doxycyclin salt of Sucrose-octa-O-Sulphonic Acid

Analogously to Example 23, using 18.4004 g (39.7838 mmol) Doxycyclin monohydrate in 400 ml 0.1M HCl (pH 1.16) and 6.5185 g (5.6262 mmol) sucrose-octa-O-sulphonic acid-Na₈, 8 aq., in 300 ml ion-exchanged water.
Yield: 21,1633 g

### EXAMPLE 28

### Cinnarizine salt of Sucrose-octa-O-Sulphonic Acid

Analogously to Example 23, using 7.3713 g (20.0035 mmol) Cinnarizine in 400 ml 1M HCl (pH 0.05) and 2.9450 g (2.5419 mmol) sucrose-octa-O-sulphonic acid-Na₈, 8 aq., in 250 ml ion-exchanged water.
Yield: 1,0216 g

### EXAMPLE 29

### Ergotamine Salt of Sucrose-octa-O-sulphonic Acid

1.0189 g (0.7758 mmol) Ergotamine Tartrate is dissolved in 400 ml 0.1M HCl (pH1.62). The compound is almost insoluble, so the solution has to be decanted. A solution of 0.1302 g (0.1124 mmol) sucrose-octa-O-sulphonic acid-Na₈, 8 aq., in 100 ml ion-exchanged water is added, which results in precipitation. The reaction mixture is filtered and vacuum dried at lTorr/25°C/20h/ Sicapent®.
Yield: 0.5549 g

### EXAMPLE 30

### Furosemide salt of Sucrose-octa-O-Sulphonic Acid

13.2759 g (40.1363 mmol) Furosemide is dissolved in 400 ml methanol (pH 3.82) and by addition of 5.8505 g (5.0496 mmol) sucrose-octa-O-sulphonic acid-Na₈, 8 aq., dissolved in 300 ml ion-exchanged water, precipitation takes place. The reaction mixture is filtered and vacuum dried at 1Torr/25°C/20h/Sicapent®.
Yield: 9.9696 g

### EXAMPLE 31

### Cyproheptadiene salt of Sucrose-octa-O-Sulphonic Acid

Analogously to Example 30, using 0.4260 g (1.2141 mmol) Cyproheptadiene·HCl sesquihydrate in 100 ml methanol (pH 4.80) and 0.1850 g (0.1597 mmol) sucrose-octa-O-sulphonic acid-Na₈, 8 aq., in 100 ml ion-exchanged water.

### EXAMPLE 32

### Carbamazepine salt of Sucrose-octa-O-Sulphonic Acid

9.4229 g (39.8836 mmol) Carbamazepine is dissolved in 400 ml methanol (pH 7.70). The solution is adjusted to pH 4.00 with 1M HCl (pH 2.03). A solution of 5.7700 g (4.9801 mmol) sucrose-octa-O-sulphonic acid-Na₈, 8 aq., in 300 ml ion-exchanged water is added, which results in precipitation. The reaction mixture is filtered and vacuum dried at 1Torr/25°C/20h/Sicapent®.

### EXAMPLE 33

### Indomethacine salt of Sucrose-octa-O-Sulphonic Acid

Analogously to Example 32, using 14.3550 g (40.1191 mmol) Indomethacine in 400 ml absolute ethanol (pH 5.02) and 5.8419 g (5.0422 mmol) sucrose-octa-O-sulphonic acid-Na₈, 8 aq., in 300 ml ion-exchanged water.
Yield: 1.3736 g

### EXAMPLE 34

### Amitriptyline salt of Phytic Acid

2.0466 g (6.5201 mmol) Amitriptyline·HCl is dissolved in 100 ml ion-exchanged water (pH 3.89) and by addition of 100 ml 0.01082M Phytic acid, precipitation takes place. The reaction mixture is filtered and vacuum dried at 1Torr/25°C/20h/Sicapent®.

### EXAMPLE 35

### Chlordiazepoxid salt of Phytic Acid

Analogously to Example 34, using 2.1999 g (6.5419 mmol) Chlordiazepoxid·HCl in 200 ml ion-exchanged water (pH 3.34) and 100 ml 0.01082M phytic acid.
Yield: 1.2698 g

### EXAMPLE 36

### Chlorpromazine salt of Phytic Acid

Analogously to Example 34, using 2.3396 g (6.5832 mmol) Chlorpromazine·HCl in 100 ml ion-exchanged water (pH 4.84) and 100 ml 0.01082M phytic acid.
Yield: 0.9340 g

### EXAMPLE 37

### Cyclobenzaprine salt of Phytic Acid

Analogously to Example 34, using 2.0401 g (6.5421 mmol) Cyclobenzaprine·HCl in 100 ml ion-exchanged water (pH 4.54) and 100 ml 0.01082M phytic acid.

### EXAMPLE 38

### Diltiazem salt of Phytic Acid

Analogously to Example 34, using 2.9578 g (6.5586 mmol) Diltiazem·HCl in 100 ml ion-exchanged water (pH 4.84) and 100 ml 0.01082M phytic acid.
Yield: 1.5777 g

### EXAMPLE 39

### Noscapine salt of Phytic Acid

Analogously to Example 34, using 2.9277 g (6.5083 mmol) Noscapine·HCl in 100 ml ion-exchanged water (pH 4.46) and 100 ml 0.01082M phytic acid.
Yield: 2.4057 g

### EXAMPLE 40

### Vancomycin salt of Phytic Acid

Analogously to Example 34, using 9.6587 g (6.5008 mmol) vancomycin·HCl in 200 ml ion-exchanged water (pH 3.64) and 100 ml 0.01082M phytic acid.
Yield: 6.4126 g

### EXAMPLE 41

### Diphenhydramine salt of Phytic Acid

1.8906 g (6.4780 mmol) Diphenhydramine·HCl is dissolved in 100 ml ion-exchanged water (pH 6.11). The solution is adjusted to pH 4.00 with 1M HCl (pH 4.01) and by addition of 100 ml 0.01082M phytic acid, precipitation takes place. The reaction mixture is filtered and vacuum dried at 1Torr/25°C/20h/Sicapent®.

### EXAMPLE 42

### Quinidine salt of Phytic Acid

Analogously to Example 41, using 4.8460 g (6.4879 mmol) Quinidine Sulphate in 600 ml ion-exchanged water (pH 6.68) and 100 ml 0.01082M phytic acid.
Yield: 1.4377 g

### EXAMPLE 43

### Tobramycin salt of Phytic Acid

Analogously to Example 41, using 3.0606 g (6.5462 mmol) Tobramycin in 100 ml ion-exchanged water (pH 10.51) and 100 ml 0.01082M phytic acid.
Yield: 0.9609 g

### EXAMPLE 44

### Verapamil salt of Phytic Acid

Analogously to Example 41, using 3.1870 g (6.4903 mmol) Verapamil·HCl in 200 ml ion-exchanged water (pH 6.90) and 100 ml 0.01082M phytic acid.

### EXAMPLE 45

### Analysis of sucrose-octa-O-sulphonic acid salts

The following drug substances and the Sucrose-octa-O-sulphonic acid salts of these substances were analysed as described below.

The Sucrose-octa-O-sulphonic acid salt of the active substance was dissolved in a 1% sodium chloride solution and analysed by HPLC, using the parameters below:
- Mobile phase:: Ammonium sulphate solution in water, 132mg/ml.
- Detection:: Refractive index detector.
- Column:: 5% phenyl 95% methylpolysilane chemically bound to silica gel, 3.9mm x 30cm.

The content of the active substances was measured by UV when possible, at a suitable UVₘₐₓ of the active substance.

The Sucrose-octa-O-sulphonic acid salt of the active substance was analysed by DSC. The melting point or destruction point of the active substance was determined. The destruction temperature of the Sucrose-octa-O-sulphonic acid salt of the substance was found, and it was shown that the melting or destruction temperature of the active substance could not be recognized in the Sucrose-octa-O-sulphonic acid salt. Additionally it was shown that the destruction temperature of Sucrose-octa-O-sulphonic acid sodium salt was different from the destruction temperature of the Sucrose-octa-O-sulphonic acid salt of the active substances.

Sucrose-octa-O-sulphonic acid sodium salt itself has a destruction temperature of about 125°C.

### Bacitracin-SOS:

Bacitracin: Not analysed, due to no chromophoric groups being present.

DSC: The Sucrose-octa-O-sulphonic acid salt of bacitracin has no melting point, but starts to decompose at about 150°C. The destruction temperature of Sucrose-octa-O-sulphonic acid sodium salt is about 125°C, this indicates that Sucrose-octa-O-sulphonic acid and bacitracin precipitate as a salt.

### Cyclobenzaprine-SOS:

Cyclobenzaprine: 32% calculated with reference to the non-dried substance.
DSC: Melting point of cyclobenzaprine was found to be 214.9°C.
The Sucrose-octa-O-sulphonic acid salt of cyclobenzaprine has no melting point, but the substance starts to decompose at about 200°C.
No endotherm was seen at 214.9°C, showing that cyclobenzaprine was not present as crystals in the mixture.
This indicates that Sucrose-octa-O-sulphonic acid and cyclobenzaprine precipitate as a salt.

### Diltiazem-SOS:

Diltiazem: 79% calculated with reference to the non-dried substance.
DSC: Melting point of diltiazem was found to be 207.6°C. The Sucrose-octa-O-sulphonic acid salt of diltiazem has no melting point, but the substance starts to decompose at 170°C.
No endotherm was seen at 207.6°C, showing that diltiazem was not present as crystals in the mixture.
This indicates that Sucrose-octa-O-sulphonic acid and diltiazem precipitate as a salt.
Water: 3.9%

### Erythromycin-SOS:

Erythromycin: Not analysed, due to no chromophoric groups being present.
DSC: Erythromycin hydrated crystals melt at 135-140°C, resolidify and melt again at 190-193°C.
The Sucrose-octa-O-sulphonic acid salt of erythromycin has no melting point, but the substance starts to decompose at 200°C.
A small exotherm and endotherm is seen at 130-140°C showing that a small amount of erythromycin might be present as crystals in the salt. However, most of the erythromycin is not present as crystals in the mixture. This indicates that Sucrose-octa-O-sulphonic acid and erythromycin precipitate as a salt.

### Nortriptyline-SOS:

SOS: 38% calculated with reference to the non-dried substance.
Nortriptyline: 43% calculated with reference to the non-dried substance.
DSC: Melting point of nortriptyline was found to be 213.3°C.
The Sucrose-octa-O-sulphonic acid salt of nortriptyline has no melting point, but starts to decompose at 190°C. No endotherm was seen at 213.3°C, showing that nortriptyline was not present as crystals in the mixture.
This indicates that Sucrose-octa-O-sulphonic acid and nortriptyline precipitate as a salt.

### Noscapine-SOS:

Noscapine: 87% calculated with reference to the non-dried substance.
DSC: Melting point of noscapine was found to be 203.7°C. The Sucrose-octa-O-sulphonic acid salt of noscapine has no melting point, but starts to decompose at 200°C. No endotherm was seen at 203.7°C, showing that noscapine was not present as crystals in the mixture.
This indicates that Sucrose-octa-O-sulphonic acid and noscapine precipitate as a salt.

### Polymyxin-SOS:

SOS: 37.8% calculated with reference to the non-dried substance.
Polymyxin: Not analysed, due to no chromophoric groups being present.
DSC: Polymyxin starts to decompose at 125°C.
The Sucrose-octa-O-sulphonic acid salt of polymyxin has no melting point, but does not start to decompose before 160°C.
This indicates that polymyxin is not present as crystals in the mixture and that Sucrose-octa-O-sulphonic acid and polymyxin precipitate as a salt.

### Quinidine-SOS:

SOS: 39%
Quinidine: 69%
DSC: Quinidine melts at 170°C, recrystallizes and melts again at 180°C.
The Sucrose-octa-O-sulphonic acid salt of quinidine has no melting point, but starts to decompose at 175°C. No endotherm was seen at 170 or 180°C, showing that quinidine was not present as crystals in the mixture. This indicates that Sucrose-octa-O-sulphonic acid and quinidine precipitate as a salt.

### Vancomycin-SOS:

Vancomycin: Not analysed, due to no chromophoric groups being present.
DSC: Vancomycin starts to decompose at 125°C.
The Sucrose-octa-O-sulphonic acid salt of vancomycin has no melting point, but does not start to decompose before 135°C.
This indicates that vancomycin is not present as crystals in the mixture and that Sucrose-octa-O-sulphonic acid and vancomycin precipitate as a salt.

### Verapamil-SOS:

Verapamil: 93% calculated with reference to the non-dried substance.
DSC: Melting point of verapamil was found to be 141.2°C. The Sucrose-octa-O-sulphonic acid salt of verapamili has no melting point, but starts to decompose instead. No endotherm was seen at 141.2°C, showing that verapamil was not present as crystals in the mixture.
This indicates that verapamil is not present as crystals in the mixture and that Sucrose-octa-O-sulphonic acid and verapamil precipitate as a salt.

### Benztropine-SOS:

SOS: 19.1 % calculated with reference to the non-dried substance.
Benztropine: 54 % calculated with reference to the non-dried substance.

### EXAMPLE 46

### Analysis of Phytic acid salts

The drug substance and the phytic acid salt of the substance were analysed as described in Examples 120-124.

### Diltiazem-phytic acid:

The content of diltiazem in the salt was found to be 82 %.
UV: Comparing the UV profile of diltiazem phytic acid iwith that of diltiazem it was concluded that diltiazem is one of the components of the salt (phytic acid has only negligible absorbance).
IR: The infrared absorption spectra of diltiazem, phytic acid and diltiazem phytic acid indicate that diltiazem and phytic acid are present in the salt.
DSC: In the thermogram of diltiazem phytic acid the decomposition starts at 200°C. The destruction point of phytic acid 125-130°C, and the destruction point of diltiazem at 187-188°C is absent. This indicates that phytic acid and diltiazem precipitate as a salt.

### Quinidine-phytic acid:

The content of quinidine in the salt was found to be 75 %.
UV: Comparing the UV profile of quinidine phytic acid iwith that of quinidine it was concluded that quinidine is one of the components of the salt (phytic acid has only negligible absorbance).
IR: The infrared absorption spectra of quinidine, phytic acid and quinidine phytic acid indicate that quinidine is present in the salt.
DSC: In the thermogram of quinidine phytic acid the decomposition starts at 140°C. The destruction point of phytic acid 125-130°C, and the destruction point of quinidine expected at 177°C is absent. This indicates that phytic acid and quinidine precipitate as a salt.

### Noscapine-phytic acid:

The content of noscapine in the salt was found to be 84 %.
UV: Comparing the UV profile of noscapine phytic acid with that of noscapine it was concluded that noscapine
is one of the components of the salt (phytic acid has only negligible absorbance).
IR: The infrared absorption spectra of noscapine, phytic acid and noscapine phytic acid indicate that noscapine is present in the salt.
DSC: In the thermogram of noscapine phytic acid, melting of noscapine is seen at 175°C. (Expected melting point of noscapine is 176°C). The substance starts to decompose at 180-190°C.

### Chlordiazepoxide-phytic acid:

The content of chlordiazepoxide in the salt was found to be 94 %.
UV: Comparing the UV profile of chlordiazepoxide phytic acid with that of chlordiazepoxide it was concluded that chlordiazepoxide is one of the components of the salt (phytic acid has only negligible absorbance).
IR: The infrared absorption spectra of chlordiazepoxide, phytic acid and chlordiazepoxide phytic acid indicate that chlordiazepoxide is present in the salt.
DSC: In the thermogram of the chlordiazepoxide phytic acid salt, decomposition starts at 200°C. Expected melting point of chlordiazepoxide HCl is 213°C. The destruction point of phytic acid at 125-130°C and the melting endotherm of chlordiazepoxide are absent. This indicates that phytic acid and chlordiazepoxide precipitate as a salt.

### EXAMPLE 47

### Investigation of Oesophageal Irritatin in the Cat

The ulcergogenic properties of drugs such as doxycycline are well documented (see for example Delphre et al. Digestive Diseases and Sciences 34: 797-800 (1989)).

The following test was carried out to compare the local ulcerogenic effects of doxycycline in four different administration forms.

Four groups, each of 4 cats, received doxycycline carrageenate, doxycycline monohydrate, doxycycline hyclate and doxycycline sucrose octasulfate, respectively (one tablet/cat).

The animals were anaesthesized and the tablet was placed in the oesophagus approximately 5 cm below the upper sphincter. The mucosa was inspected for lesions, immediately before and after the administration of the tablet, using an endoscope.

The animals were kept constantly anaesthetized and were killed 8 hours after tablet administration.

The oesophagus was exposed and external aspects were inspected. The oesophagus was opened in situ, the mucosa inspected and photographed.

The degree of disintegration of the tablets and any changes of the oesophagus were noted.

Two samples of oesophagus were collected, one from the site of tablet and one anterior to the site of tablet. The samples were prepared for histologic examination. The microscopic changes were recorded.

None of the tablets were completely disintegrated. The doxycycline monohydrate tablets were almost intact, the doxycycline carrageenate tablets were disintegrated to an intermediate stage, whereas the doxycycline hyclate and doxycycline sucrose-octasuffate tablets were mostly disintegrated.

As the only macroscopic finding, a dry area was observed on the oesophageal mucosa in five cats.

Microscopically no changes were seen in the sample taken anterior to the site of the tablet. In the sample from the site of tablet from cats receiving doxycycline monohydrate or doxycycline sucrose octasulfate, no changes were observed.

In one cat receiving doxycycline carrageenate, thinning of epithelium characterised by desquamation of outer layer and slight focal subepithelial and epithelial accumulation of primary neutrophils were observed.

In all four cats receiving doxycycline hyclate, moderate swelling and vacuolation of epithelial cells were observed. The basal layer remained intact in two of the cats whereas in the other two the vacuolation was present in the cells of the basal layer as well. Minimal focal epithelial accumulation of neutrophils was found in one cat.

This test showed that doxycycline monohydrate and doxycycline sucrose octasulfate tablets did not cause any damage to the oesophageal mucosa whereas minor lesions were seen in one out of four cats receiving doxycycline carrageenate and four of four cats receiving doxycycline hyclate.

## Claims

1. A water-insoluble or poorly water-soluble therapeutic compound being a mono- to octa-saccharide acid salt of a biologically active organic compound, other than a sucrose-octa-O-sulphonic acid salt of an aminoglycoside.

2. A compound as claimed in claim 1 which is insoluble or poorly soluble in deionized water.

3. A compound as claimed in either of the preceding claims wherein said acid is a poly-O-sulphonic acid.

4. A compound as claimed in any one of the preceding claims wherein said acid is a disaccharide poly-O-sulphonic acid.

5. A compound as claimed in any one of the preceding claims wherein said acid is sucrose-octa-O-sulphonic acid.

6. A compound as claimed in any one of the preceding claims further containing a physiologically tolerable counterion.

7. A compound as claimed in claim 6 wherein said counterion is selected from alkali metal, alkaline earth metal, ammonium and aluminium ions.

8. A compound as claimed in claim 7 wherein said counterion is an aluminium ion.

9. A compound as claimed in any one of the preceding claims wherein said organic compound is a basic nitrogen atom containing compound.

10. A compound as claimed in claim 9 wherein said organic compound contains a plurality of protonatable nitrogen atoms.

11. A compound as claimed in any one of the preceding claims wherein said organic compound is selected from the group consisting of antibacterials, antivirals, antimycotics, anti-amoebics, antiallergics, cardioprotectives, analgesics, anxiolytics, sedatives, hypnotics, anti-migraine agents, anti-motion sickness agents, anti-emetics, adrenergics, antispasmodics, muscle relaxants, neuroleptics, antidepressants, anticholinergics, antihistamines, anti-anorexics, and alkaloids.

12. A compound as claimed in any one of the preceding claims wherein said organic compound is an antibacterial.

13. A compound as claimed in claim 12 wherein said organic compound is an aminoglycoside, a tetracyclin, a polypeptide, a glycopeptide or a macrolide.

14. A compound as claimed in claim 12 wherein said organic compound is a tetracyclin.

15. A compound as claimed in claim 12 wherein said organic compound is selected from the group consisting of doxycyclin, diltiazam, cyclobenzaprine, bacitracin, noscapine, erythromycin, polymyxin, vancomycin, nortriptyline, quinidine, ergotamine, benztropine, verapamil, flunarizine and imipramine.

16. A compound as claimed in claim 1 wherein said organic compound is selected from pindolol, diclofenac, desipramine amitriptyline, chlordiazepoxid, chlorpromazine, diphenhydramine, tobramycin, cinnarizine, furosemide, cyproheptadiene, carbamazepine, indomethacine and propranolol.

17. A compound as claimed in any one of claims 14 to 16 being a sucrose-octa-O-sulphonic acid salt of a said organic compound.

18. A compound as claimed in any one of claims 1 to 16 wherein said acid is a polysulphonic or polyphosphonic acid.

19. A compound as claimed in claim 1 being the sucrose-octa-O-sulphonic acid salt of doxycyclin.

20. A compound as claimed in any one of claims 1 to 19 for use as a therapeutic agent.

21. A pharmaceutical composition comprising a biologically active organic compound together with at least one physiologically acceptable carrier or excipient, characterized in that said compound, which is other than a sucrose-octa-O-sulphonic acid salt of an aminoglycoside, is present in the form of a water-insoluble or poorly water soluble salt with a mono- to octa-saccharide acid.

22. Use of an effective amount of a biologically active organic compound in the manufacture of a medicament for combatting a condition responsive to said compound, characterized in that said compound is administered as a water-insoluble or poorly water soluble salt with a mono- to octa-saccharide acid, with the proviso that said salt is other than a sucrose-octa-O-sulphonic acid salt of an aminoglycoside.

23. Use as claimed in claim 22 wherein said salt is administered perorally.

24. Use as claimed in claim 22 wherein said salt is administered topically to a mucous membrane.

## Patentansprüche

1. Wasserunlösliche oder gering wasserlösliche therapeutische Verbindung, welche ein Mono- bis Octa-saccharidsäuresalz einer biologisch wirksamen organischen Verbindung ist, außer einem Saccharose-octa-O-sulfonsäuresalz eines Aminoglycosids.

2. Verbindung nach Anspruch 1, welche in deionisiertem Wasser unlöslich oder gering löslich ist.

3. Verbindung nach einem der vorhergehenden Ansprüche, worin die Säure eine Poly-O-sulfonsäure ist.

4. Verbindung nach einem der vorhergehenden Ansprüche, worin die Säure eine Disaccharid-poly-O-sulfonsäure ist.

5. Verbindung nach einem der vorhergehenden Ansprüche, worin die Säure Saccharose-octa-O-sulfonsäure ist.

6. Verbindung nach einem der vorhergehenden Ansprüche, weiterin enthaltend ein physiologisch tolerierbares Gegenion.

7. Verbindung nach Anspruch 6, worin das Gegenion ausgewählt ist aus Alkalimetall-, Erdalkalimetall-, Ammonium- und Aluminiumionen.

8. Verbindung nach Anspruch 7, worin das Gegenion ein Aluminiumion ist.

9. Verbindung nach einem der vorhergehenden Ansprüche, worin die organische Verbindung eine ein basisches Stickstoffatom enthaltende Verbindung ist.

10. Verbindung nach Anspruch 9, worin die organische Verbindung mehrere protonierbare Stickstoffatome enthält.

11. Verbindung nach einem der vorhergehenden Ansprüche, worin die organische Verbindung ausgewählt ist aus der Gruppe, bestehend aus Bakterien hemmenden Mitteln, Viren hemmenden Mitteln, Antimycotika, Antiamoebica, Antiallergika, herzschützenden Mitteln, Analgetika, Anxiolytika, Beruhigungsmitteln, Hypnotika, Antimigränemitteln, Mitteln gegen Reisekrankheit, Antiemetica, Adrenergica, Antispasmodica, Muskelrelaxantien, Neuroleptica, Antidepressiva, Anticholinergica, Antihystaminen, Antianorexica und Alkaloiden.

12. Verbindung nach einem der vorhergehenden Ansprüche, worin die organische Verbindung ein Bakterien hemmendes Mittel ist.

13. Verbindung nach Anspruch 12, worin die organische Verbindung ein Aminoglycosid, ein Tetracyclin, ein Polypeptid, ein Glycopeptid oder ein Macrolid ist.

14. Verbindung nach Anspruch 12, worin die organische Verbindung ein Tetracyclin ist.

15. Verbindung nach Anspruch 12, worin die organische Verbindung ausgewählt ist aus der Gruppe, bestehend aus Doxycyclin, Diltiazam, Cyclobenzaprin, Bacitracin, Noscapin, Erythromycin, Polymyxin, Vancomycin, Nortriptylin, Chinidin, Ergotamin, Benztropin, Verapamil, Flunarizin und Imipramin.

16. Verbindung nach Anspruch 1, worin die organische Verbindung ausgewählt ist aus Pindolol, Diclofenac, Desipramin, Amitriptylin, Chlordiazepoxid, Chlorpromazin, Diphenhydramin, Tobramycin, Cinnarizin, Furosemid, Cyproheptadien, Carbamazepin, Indomethacin und Propanolol.

17. Verbindung nach einem der Ansprüche 14 bis 16, welche ein Saccharose-octa-O-sulfonsäuresalz der organischen Verbindung ist.

18. Verbindung nach einem der Ansprüche 1 bis 16, worin die Säure eine Polysulfon- oder Polyphosphonsäure ist.

19. Verbindung nach Anspruch 1, welche das Saccharose-octa-O-sulfonsäuresalz von Doxycyclin ist.

20. Verbindung nach einem der Ansprüche 1 bis 19 zur Verwendung als therapeutisches Mittel.

21. Pharmazeutische Zusammensetzung, umfassend eine biologisch wirksame organische Verbindung zusammen mit mindestens einem physiologisch annehmbaren Träger oder Hilfsstoff,
dadurch gekennzeichnet,
daß die Verbindung, die kein Saccharose-octa-O-sulfonsäuresalz eines Aminoglycosids ist, in Form eines wasserunlöslichen oder gering wasserlöslichen Salzes mit einer Mono- bis Octa-Saccharidsäure vorliegt.

22. Verwendung einer wirksamen Menge einer biologisch wirksamen organischen Verbindung bei der Herstellung eines Arzneimittels zur Bekämpfung eines auf die Verbindung ansprechenden Krankheitszustandes,
dadurch gekennzeichnet,
daß die Verbindung als wasserunlösliches oder gering wasserlösliches Salz mit einer Mono- bis Octa-Saccharidsäure verabreicht wird, mit der Maßgabe, daß das Salz kein Saccharose-octa-O-sulfonsäuresalz eines Aminoglycosids ist.

23. Verwendung nach Anspruch 22, worin das Salz peroral verabreicht wird.

24. Verwendung nach Anpruch 22, worin das Salz topisch auf eine Schleimhautmembran verabreicht wird.

## Revendications

1. Composé thérapeutique insoluble dans l'eau ou faiblement soluble dans l'eau, consistant en un sel de mono à octa-acide saccharidique d'un composé organique biologiquement actif autre qu'un sel d'acide saccharose-octa-O-sulfonique d'un aminoglycoside.

2. Composé selon la revendication 1, qui est insoluble ou faiblement soluble dans de l'eau désionisée.

3. Composé selon l'une quelconque des revendications précédentes, dans lequel ledit acide est un acide poly-O-sulfonique.

4. Composé selon l'une quelconque des revendications précédentes, dans lequel ledit acide est un acide disaccharide poly-O-sulfonique.

5. Composé selon l'une quelconque des revendications précédentes, dans lequel ledit acide est l'acide saccharose-octa-O-sulfonique.

6. Composé selon l'une quelconque des revendications précédentes, contenant en outre un contre-ion physiologiquement acceptable.

7. Composé selon la revendication 6, dans lequel ledit contre-ion est choisi parmi les ions de métal alcalin, de métal alcalino-terreux, ammonium et aluminium.

8. Composé selon la revendication 7, dans lequel ledit contre-ion est un ion aluminium.

9. Composé selon l'une quelconque des revendications précédentes, dans lequel ledit composé organique est un composé à atome d'azote basique.

10. Composé selon la revendication 9, dans lequel ledit composé organique contient plusieurs atomes d'azote protonables.

11. Composé selon l'une quelconque des revendications précédentes, dans lequel ledit composé organique est choisi parmi les agents antibactériens, antiviraux, antimycosiques, anti-amibiens, anti-allergiques, cardioprotecteurs, analgésiques, anxiolytiques, sédatifs, hypnotiques, anti-migraineux, anti-maladie des transports, antiémétiques, adrénergiques, antispasmodiques, relaxants musculaires, neuroleptiques, antidépresseurs, anticholinergiques, antihistaminiques, antianorexiques, et les alcaloïdes.

12. Composé selon l'une quelconque des revendications précédentes, dans lequel ledit composé organique est un agent antibactérien.

13. Composé selon la revendication 12, dans lequel ledit composé organique est un aminoglycoside, une tétracycline, un polypeptide, un glycopeptide ou un macrolide.

14. Composé selon la revendication 12, dans lequel ledit composé organique est une tétracycline.

15. Composé selon la revendication 12, dans lequel ledit composé organique est choisi parmi la doxycycline, le diltiazem, la cyclobenzaprine, la bacitracine, la noscapine, l'érythromycine, la polymyxine, la vancomycine, la nortriptyline, la quinidine, l'ergotamine, la benztropine, le vérapamil, la flunarizine et l'imipramine.

16. Composé selon la revendication 1, dans lequel ledit composé organique est choisi parmi le pindolol, le diclofénac, la désipramine, l'amitriptyline, le chlordiazépoxyde, la chlorpromazine, la diphénhydramine, la tobramycine, la cinnarizine, le furosémide, le cyproheptadiène, la carbamazépine, l'indométhacine et le propranolol.

17. Composé selon l'une quelconque des revendications 14 à 16, consistant en un sel d'acide saccharose-octa-O-sulfonique et dudit composé organique.

18. Composé selon l'une quelconque des revendications 1 à 16, dans lequel ledit acide est un acide polysulfonique ou polyphosphonique.

19. Composé selon la revendication 1, consistant en le sel d'acide saccharose-octa-O-sulfonique de la doxycycline.

20. Composé selon l'une quelconque des revendications 1 à 19, destiné à être employé comme agent thérapeutique.

21. Composition pharmaceutique comprenant un composé organique biologiquement actif en association avec au moins un vecteur ou excipient physiologiquement acceptable, caractérisée en ce que ledit composé, qui est autre qu'un sel d'acide saccharose-octa-O-sulfonique d'un aminoglycoside, est présent sous forme d'un sel insoluble dans l'eau ou peu soluble dans l'eau, avec un mono à octaacide saccharidique.

22. Utilisation d'une quantité efficace d'un composé organique biologiquement actif pour la fabrication d'un médicament pour combattre un état sensible audit composé, caractérisée en ce que ledit composé est administré sous forme d'un sel insoluble dans l'eau ou peu soluble dans l'eau avec un mono à octaacide saccharidique, à condition que ledit sel soit autre qu'un sel d'acide saccharose octa-O-sulfonique d'un aminoglycoside.

23. Utilisation selon la revendication 22, dans lequel ledit sel est administré par voie orale.

24. Utilisation selon la revendication 22, dans laquelle ledit sel est administré de manière topique sur une membrane muqueuse.
